# EUROPEAN PATENT APPLICATION

(11) **EP 4 796 159 A2**
(43) Date of publication of application: **26.08.2026**
(21) Application number: 26154746.7
(22) Date of filing: 28.01.2026
(51) Int. Cl.: A61M 60/13, A61M 60/221, A61M 60/865, A61M 60/90

(54) **BLOOD PUMP HAVING A PUNCTURE RESISTANT CATHETER**

(30) Priority: 29.01.2025 US 202563750829 P
(71) Applicant: Abiomed, Inc., Danvers, MA 01923 (US)
(72) Inventor: SHEILS, Christopher, Danvers, 01923 (US)
(74) Representative: Wittmer, Maximilian

(57) **Abstract**

A puncture-resistant catheter and/or a puncture-resistant sleeve for a catheter may be provided. The puncture-resistant catheter may have a catheter portion and a puncture-resistant portion. The puncture-resistant portion may be made of a material which is resistant to needles and/or sharp objects. The puncture-resistant portion may be a layer on the outside of the catheter or may be a sleeve attachable to a repositioning unit.

## Description

### CROSS-REFERENCE

The present application claims priority to U.S. Provisional Patent Application No. 63/750,829, filed January 29, 2025, the contents of which are incorporated by reference herein in its entirety.

### TECHNICAL FIELD

Embodiments disclosed herein relate to medical devices, such as catheters and catheter-based devices.

### BACKGROUND

Currently, percutaneous mechanical support devices are leveraged for a variety of clinical indications. Such support devices may comprise, but are not limited to, an Impella^{®} pump, an Extracorporeal Membrane Oxygenation (ECMO) pump, and a balloon pump. The Impella^{®} pump may further comprise an Impella CP^{®} pump, Impella 5.5^{®} pump, Impella RP^{®} pump, and/or an Impella RP Flex^{®} pump, all of which are by Abiomed, Inc. of Danvers, Mass. Most often they are inserted into a patient percutaneously through a single access point (e.g., radial access, femoral access, axillary access) while other procedures, such as, for example, percutaneous coronary intervention (PCI) are performed through a second access point, such as a contralateral femoral or radial access point. The use of multiple devices on a patient at the same time therefore often requires multiple access sites which presents several challenges.

Blood pump assemblies, such as intracardiac or intravascular blood pumps may be introduced in the heart to deliver blood from the heart into an artery. Such mechanical circulatory support devices are often introduced to support the function of the heart after a patient suffers a cardiac episode. One such class of devices is the set of devices known as the "Impella" heart pump. Some blood pump assemblies may be introduced percutaneously through the vascular system during a cardiac procedure. Specifically, blood pump assemblies can be inserted via a catheterization procedure through the femoral artery or the axillary/subclavian artery, into the ascending aorta, across the valve and into the left ventricle. The inserted blood pump assembly may be configured to pull blood from the left ventricle of the heart through a cannula and expels the blood into the aorta. A blood pump assembly may also be configured to pull blood from the inferior vena cava and to expel blood into the pulmonary artery. Some mechanical circulatory support devices are powered by an on-board motor, while others are powered by an external motor and a drive cable.

### SUMMARY OF INVENTION

In certain aspects, a blood pump system may be provided. The blood pump system may include a catheter connected to the blood pump system, the catheter having a longitudinal axis and an outer wall extending circumferentially around and continuously along the longitudinal axis. The blood pump system may include a repositioning unit. The repositioning unit may have a proximal portion and a distal portion. At least a portion of the distal portion may be configured to be implanted into a patient's vasculature. The blood pump system may include a tubular body with a distal end and a proximal end. The proximal end may be connected to the distal portion of the repositioning unit. The tubular body may extend along at least a portion of the longitudinal axis of the catheter. The tubular body may be configured to surround at least a portion of the outer wall of the catheter.

The tubular body may be made of a material configured to be puncture resistant. The proximal end of the tubular body may be removably attached to the distal portion of the repositioning unit. The tubular body may be integrally formed with the distal portion of the repositioning unit. The tubular body may surround the entire circumference of the outer wall of the catheter. The tubular body may include a slot parallel to the longitudinal axis of the catheter. The slot may extend along at least a portion of the tubular body. The slot may extend along the entire longitudinal length of the tubular body. The tubular body may have a constant outer diameter. The tubular body may have an outer diameter that varies along the longitudinal axis of the catheter.

In certain aspects, an accessory for use with a blood pump system may be provided. The accessory may include a repositioning unit comprising a proximal portion and a distal portion. The distal portion may be configured to be inserted into a patient's body to allow a medical device to pass through the repositioning unit into the patient's body. The medical device may extend through the proximal portion and distal portion of the repositioning unit. The accessory may include a body which extends distally from the distal portion of the repositioning unit. The proximal portion, distal portion, and body together may form a lumen being configured to allow a medical device to pass through.

The body may be configured to be made of a puncture-resistant material. The body may further include a wall. The wall may have a discontinuity extending along at least a portion of the body. The discontinuity may be a slot, the slot extending parallel to a central axis of the body.

In certain aspects, a blood pump for percutaneous insertion into a patient's blood vessel may be provided. The blood pump may include a catheter. The blood pump may include a pumping device attached to the catheter. The catheter may extend along a longitudinal axis and may have a distal end and a proximal end opposite the distal end along the longitudinal axis. The catheter may include a first layer extending continuously longitudinally along the length of the catheter between the proximal end and the distal end of the catheter. The catheter may include a second layer extending longitudinally along at least a portion of the length of the catheter between the proximal and distal end of the catheter. At least one of the first layer and the second layer may be configured to be made of a puncture-resistant material. The first layer may be made of a puncture-resistant material. The second layer may be made of a puncture-resistant material. Both the first layer and second layer may be made of a puncture-resistant material. The second layer may extend from about 2 centimeters to about 5 centimeters longitudinally along the catheter.

In certain aspects, an accessory for use with a blood pump system may be provided. The blood pump system may include a blood pump. The blood pump system may include a catheter attached to the blood pump. The blood pump system may include a repositioning unit configured to receive at least a portion of the blood pump and/or catheter and to secure the blood pump system to the patient. The accessory may include a first end configured to be attached to a portion of a repositioning unit. The accessory may include a second end. The second end may be connected to the first end by a body. The body may surround at least a portion of the catheter. The accessory may be configured such that when a puncture force is applied to the body, the body deflects the puncture force and the catheter is not punctured and/or damaged by the puncture force.

In certain aspects, an accessory for use with a blood pump system may be provided. The blood pump system may include a blood pump. The blood pump system may include a catheter attached to the blood pump. The blood pump system may include a repositioning unit configured to receive at least a portion of the blood pump and/or catheter and to secure the blood pump system to the patient. The accessory may include a first end configured to be attached to a portion of a repositioning unit. The accessory may include a second end. The second end may be connected to the first end by a body. The body may surround at least a portion of the catheter. The body may be made of a puncture-resistant material.

### BRIEF DESCRIPTION OF DRAWINGS

The foregoing and other objects and advantages will be apparent upon consideration of the following detailed description, taken in conjunction with the accompanying drawings, in which like reference characters refer to like parts throughout, and in which:
FIG. 1A shows an illustrative view of a blood pump inserted into the left ventricle;
FIG. 1B shows an illustrative view of a blood pump inserted into the right ventricle;
FIG. 2 shows an illustrative side view of a puncture-resistant catheter;
FIG. 3 shows a cross-sectional view of a puncture-resistant catheter;
FIG. 4 shows an illustrative side view of a puncture-resistant sleeve;
FIG. 5A shows a cross-sectional view of a puncture-resistant sleeve;
FIG. 5B shows an illustrative side view of a puncture-resistant sleeve; and
FIG. 6 shows a method according to one embodiment.

### DETAILED DESCRIPTION

As it is known, percutaneous blood pumps may be inserted into a patient's vasculature to assist with cardiovascular function. Such blood pumps may be catheter-based. As it is also known, surgically implanted or percutaneously introduced catheters may have access sites that are sutured. For example, the access site may include a repositioning unit and/or a sheath. In such instances, the repositioning unit and/or sheath may be sutured to secure it in position and maintain stability during support. However, during suturing of the access site, physicians may inadvertently puncture the catheter with a needle or similarly sharp object. As a result of such catheter puncture, blood may inadvertently enter the inner lumen of the catheter. As a result, blood loss may occur and/or the blood pump may be damaged as a result. Additionally, a catheter puncture may not detectable at the moment it occurs. Accordingly, the inventors have recognized a need to provide a catheter which has puncture-resistant properties to decrease the risk of a catheter puncture. For example, as disclosed herein, a catheter having at least a puncture-resistant portion may be used.

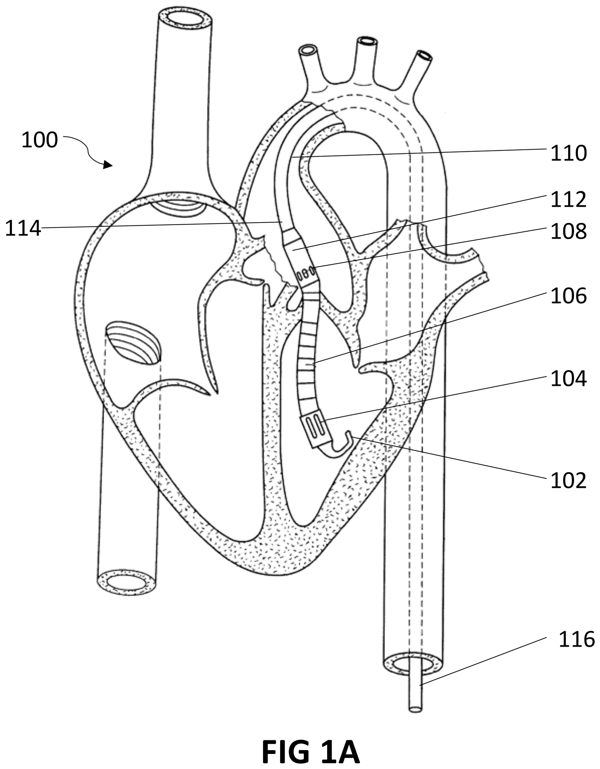

Turning to the figures, FIG. 1A shows a blood pump assembly 100 for use in a left ventricle. As shown in this view, the blood pump assembly 100 may include an atraumatic tip 102, an inlet 104, a cannula 106, an outlet 108 and a catheter 110. The blood pump assembly further may include a pump 112. The pump 112 may include an impeller (not shown) and a motor (not shown). The inlet 104 may be disposed inside of the left ventricle. The outlet 108 may be disposed inside of the aorta. An impeller (not shown) may be provided inside of the pump 112 to cause the blood flow from the inlet 104 to the outlet 108. The rotation of the impeller may be caused by the motor (not shown). At the distal end of the blood pump system 100, the atraumatic tip 102 may be arranged to facilitate insertion of the blood pump system 100 into the patient's heart without damaging surrounding tissue. Additionally, the atraumatic tip 102 may stabilize the blood pump system 100 during operation. The atraumatic tip 102 may also help keep patient anatomy inside of the left ventricle away from the inlet 104. In some embodiments, the atraumatic tip 102 may be configurable from a straight to a partially curved (as shown) configuration. In some embodiments, the pigtail may also be composed, at least in part, of a flexible material, and may have dual stiffness. In some embodiments, it should be appreciated that the blood pump assembly may not include an atraumatic tip. The catheter may have a distal end 114 and a proximal end 116. The blood pump may be placed such that it lies primarily in the ascending aorta leading to the aortic arch. The aortic valve may come to lie, in the closed state, against the pump 112 or the cannula 106 that lies substantially in the left ventricle. The blood pump may be advanced into the represented position by advancing the catheter 102, optionally employing a guide wire. In so doing, the cannula 106 may pass the aortic valve retrograde, so the blood may be sucked in through the cannula 106 and pumped into the aorta.

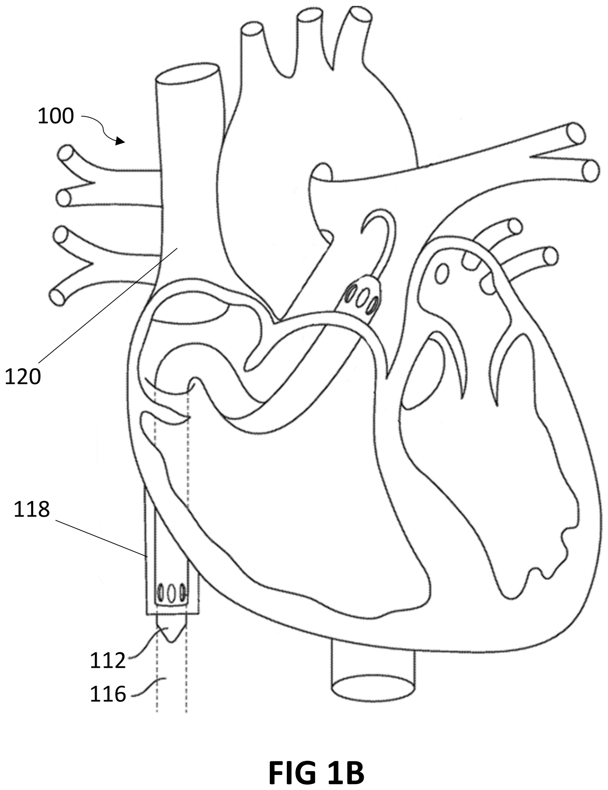

In some embodiments, the blood pump assembly 100 may be positioned in the right ventricle, as shown in FIG. 1B. As seen in the blood pump assembly of FIG. 1B, the catheter 116 may be connected to the pump 112. In some embodiments, the catheter 116 may be in the inferior vena cava 118. In other embodiments, the catheter 116 may be in the superior vena cava 120.

As will be appreciated, the use of the blood pump assembly is not restricted to the applications represented in FIGS. 1A and 1B. For example, the blood pump assembly may be inserted through other peripheral vessels, such as the subclavian artery. Alternatively, revers applications for the right ventricle may be envisioned.

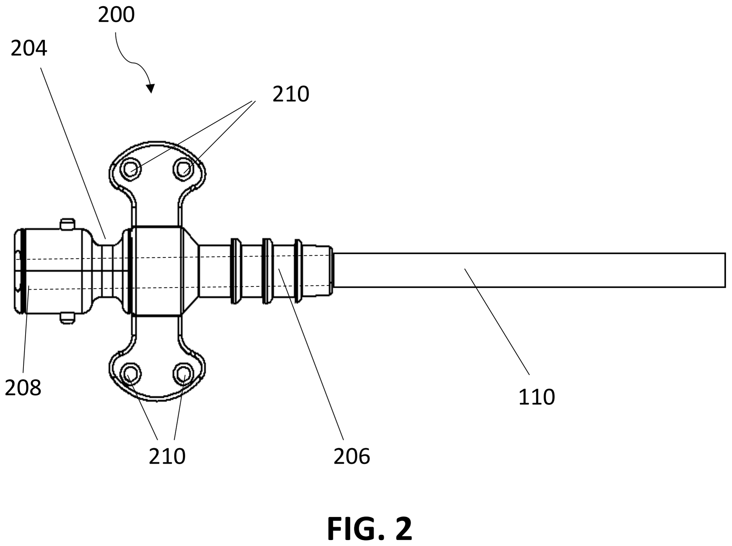

Referring now to FIG. 2, the catheter 110 of the blood pump system 100 is shown. The catheter 110 may extend from the distal end 114 to the proximal end 116. The pump 112 may be attached to the distal end 114 of the catheter 110. At least a portion of the proximal end 116 may pass through a repositioning unit 200, as shown in FIG. 2. The repositioning unit 200 may be a sheath comprising a tubular body 202. The tubular body 202 may include a proximal body 204 and a distal body 206. The tubular body 202 may include a lumen 208. At least a portion of the distal body 206 may be inserted into a patient's vasculature. The catheter 110 may be inserted through the lumen 208. The lumen 208 may extend through at least a portion of the repositioning unit 200. For example, in some embodiments, the lumen 208 may extend through both the proximal body 204 and the distal body 206. In other embodiments, the proximal body 204 may include a hemostatic valve at one end of the lumen 208. At least a portion of the catheter may enter the repositioning unit 200. Specifically, the catheter 110 may pass through the lumen 208 defined by the tubular body 202. The repositioning unit 300 may be secured to the patient via suturing or any other suitable means. When the repositioning unit 300 may be secured via suturing, the physician may suture using the holes 210. As will be appreciated, the repositioning unit 300 may sutured using any suitable method that does not include holes 210.

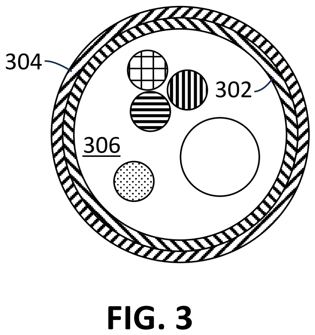

Referring now to FIG. 3, a cross-section of the catheter 110 of the blood pump assembly 100 of FIG. 1 is shown. The catheter may have an inner layer 302 and an outer layer 304. A lumen 306 may be formed inside of the inner layer 302. The inner layer 302 of the catheter extends along the entire length of the catheter, e.g., from the proximal end 116 to the distal end 114 at the pump. The inner layer 302 may be made of any suitable material for catheters. In some embodiments, the outer layer 304 may extend along the entire length of the catheter. In other embodiments, the outer layer 304 may only extend along a portion of the length of the catheter. Specifically, the outer layer 304 may extend along a portion of the catheter that is closer to the proximal end 116 and the repositioning unit 200. The outer layer 304 may be made of a puncture-resistant material. For example, the outer layer 304 may be made of a puncture-resistant polymer. The outer layer 304 may protect the inner layer 302, and thus the lumen 306, of the catheter from sharp objects that may be inserted through the repositioning unit 200. The outer layer 304 may be applied to the inner layer 302 via any suitable means, such as over-molding. In some embodiments, the inner layer 302 may be made of a puncture-resistant material. In some embodiments, the thickness of the inner layer 302 and outer layer 304 may be constant along the entire length of the catheter 110. In other embodiments, the thickness of the outer layer 304 may vary along the length of the catheter 110. For example, the thickness of the outer layer 304 may decrease moving distally along the length of the catheter 110.

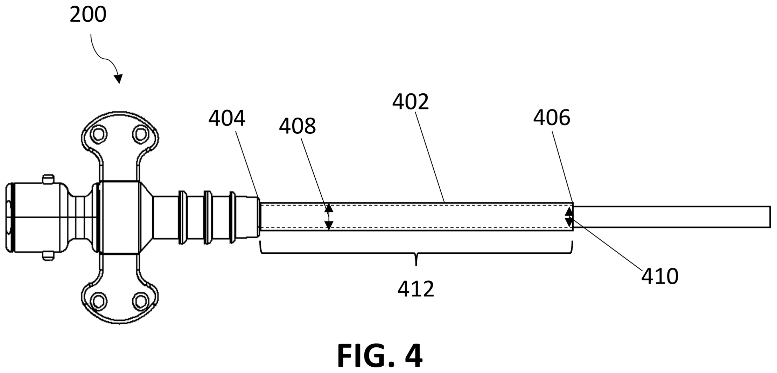

Further referring to FIG. 4, a sleeve 402 is shown. The sleeve 402 may be a tubular structure. The sleeve 402 may be circular or substantially circular as shown in FIG. 4. The sleeve 402 may have a proximal end 404 and a distal end 406. In some embodiments, the proximal end 404 may be attached to the repositioning unit 200. In other embodiments, the sleeve 402 may be over-molded with the repositioning unit 200. In other embodiments, the sleeve 402 may be removably attached to the repositioning unit 200. The distal end 406 may be located distal to the proximal end 404. In some embodiments, the sleeve 402 may be made of a material that is puncture-resistant.

In some embodiments, the sleeve 402 may have an outer diameter 408 and an inner diameter 410. In one embodiment, the outer diameter 408 may be continuous along the length of the sleeve 402. In some embodiments, the sleeve 402 may have a continuous wall around the circumference of the catheter 110. In other embodiments, the outer diameter 408 may vary along the length of the sleeve 402. For example, the outer diameter 408 may be greater at the proximal end 404 and may decrease along the length of the sleeve 402, moving toward the distal end 406. In still other embodiments, the outer diameter 408 may vary because the puncture-resistant properties may be only desired in a certain area of the catheter 110. For example, the physician may desire the puncture-resistant properties of the sleeve closer to the repositioning unit 200. In some embodiments, the blood pump assembly 100 may be provided with a plurality of sleeves 402 with varying outer diameters 408. In such embodiments, the physician may select a sleeve 402 that corresponds with the desired size of the access site.

In some embodiments, sleeve 402 may have a length 412 that extends along at least a portion of the longitudinal length of the catheter. For example, the sleeve 402 may have a length 412 between about 2 centimeters to about 5 centimeters. In other embodiments, the sleeve 402 may have a length 412 between about 3 centimeters and about 4 centimeters. As will be appreciated, the sleeve 402 may have any suitable length. For example, the sleeve 402 may have a length configured to cover the catheter in the graft but not long enough where it extends into the patient's vessel.

In some embodiments, there may be a gap between the sleeve 402 and the catheter 110. In some embodiments, the gap may be between about 0 millimeters and about 2 millimeters. In other embodiments, the gap may be between about 0 millimeters and about 1 millimeter. For example, the gap may be about 0.1 millimeters. As will be appreciated, the gap may be of any suitable size.

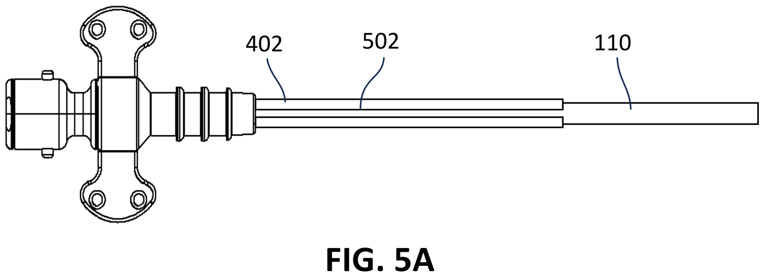

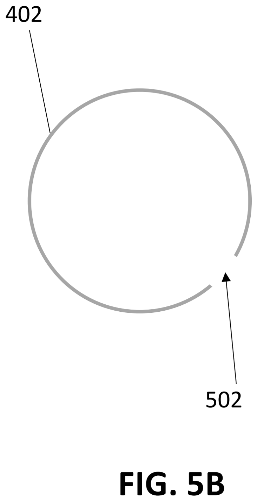

In some embodiments, as shown in FIG. 5A, the sleeve 402 may have a wall with discontinuities along the length of the sleeve 402. For example, in one embodiment, the sleeve 402 may have a slot 502. The slot 502 may continue along the entire length of the sleeve 402, as shown in FIG. 5B. In other embodiments, the slot 502 may continue along at least a portion of the sleeve 402. In other embodiments, the width of the slot 502 may vary along the length of the sleeve 402. In some embodiments, the slot 502 may be parallel to the longitudinal axis of the catheter 110. In other embodiments, the slot 502 may have a helical shape along the length of the sleeve 402. Overall, the slot 502 may allow the physician to position the sleeve 402 on the catheter 110 during the procedure if they choose. For example, in such embodiments, the physician may insert the catheter 110 into the slot 502 until the entire circumference of the sleeve 402 is around the circumference of the catheter 110. Additionally, the slot 502 may accommodate a catheter 110 with an outer diameter that is larger than the sleeve 402. Furthermore, in some embodiments, the slot 502 may also ensure that the sleeve 402 does not pinch the catheter 110 and/or obstruct the lumen inside of the catheter.

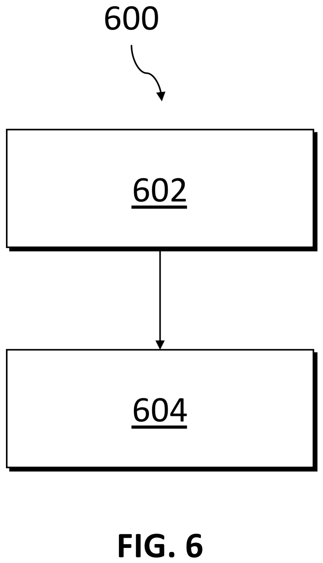

As shown in FIG. 6, a method 600 of inserting a blood pump is disclosed. As shown in this view, the method 600 may include inserting 602 a blood pump into a repositioning unit. In some embodiments, the blood pump may be connected to a catheter and at least a portion of the catheter has a puncture-resistant portion. In some embodiments, a tubular body made of a puncture-resistant material may surround the catheter. In other embodiments, the tubular body may include a slot that extends along at least a portion of the longitudinal length of the tubular body. As will be appreciated, the length of the tubular body and the slot may be any suitable length. In other embodiments, the catheter may be pressed into the slot until the tubular body circumferentially surrounds the catheter.

In other embodiments, the repositioning unit may have a proximal portion and a distal portion. In some embodiments, the distal portion may be inserted into the patient's body and allows the blood pump to pass through the repositioning unit into the patient's body. In some embodiments, the blood pump may extend through both the proximal and distal portions of the repositioning unit. In some embodiments, the repositioning unit may have a tubular body. In some embodiments, the tubular body may extend distally from the distal portion of the repositioning unit. In other embodiments, the tubular body may be made of a puncture-resistant material.

In other embodiments, the catheter may include a first layer and a second layer. The second layer may circumferentially surround the first layer of the catheter. In some embodiments, the second layer may be made of a puncture-resistant material. In some embodiments, the second layer may extend along at least a portion of the longitudinal length of the catheter. In some embodiments, the second layer may extend for about 2 centimeters to about 5 centimeters. However, as will be appreciated, the second layer may extend along any suitable length. For example, the second layer may extend for 2 centimeters. In other embodiments, the second layer may extend for 3 centimeters. In other embodiments, the second layer may extend for 4 centimeters. In other embodiments, the second layer may extend for 5 centimeters. The method 600 of FIG. 6 may also include the step of suturing 604 the repositioning unit to the patient.

Various embodiments are discussed below.

In a first embodiment, a blood pump system may be provided. The blood pump system includes: (i) a catheter connected to the blood pump system, the catheter having a longitudinal axis and an outer wall extending circumferentially around and continuously along the longitudinal axis; (ii) a repositioning unit, the repositioning unit having a proximal portion and a distal portion, at least a portion of the distal portion being configured to be implanted into a patient's vasculature; and (iii) a tubular body with a distal end and a proximal end, the proximal end connected to the distal portion of the repositioning unit; wherein the tubular body extends along at least a portion of the longitudinal axis of the catheter and is configured to surround at least a portion of the outer wall of the catheter.

In a second embodiment, based on the first embodiment, the tubular body is made of a material configured to be puncture resistant.

In a third embodiment, based on the first or second embodiment, the proximal end of the tubular body is removably attached to the distal portion of the repositioning unit.

In a fourth embodiment, based on the first or second embodiment, the tubular body is integrally formed with the distal portion of the repositioning unit.

In a fifth embodiment, based on any of the first through fourth embodiments, the tubular body surrounds the entire circumference of the outer wall of the catheter.

In a sixth embodiment, based on any of the first through fourth embodiments, the tubular body comprises a slot parallel to the longitudinal axis of the catheter.

In a seventh embodiment, based on the sixth embodiment, the slot extends along at least a portion of the tubular body.

In an eighth embodiment, based on the seventh embodiment, the slot extends along the entire longitudinal length of the tubular body.

In a ninth embodiment, based on any one of the first through eighth embodiments, the tubular body has a constant outer diameter.

In a tenth embodiment, based on any one of the first through eighth embodiments, the tubular body has an outer diameter that varies along the longitudinal axis of the catheter.

In an eleventh embodiment, an accessory for use with a blood pump system is provided, which may include: (i) a repositioning unit comprising a proximal portion and a distal portion, the distal portion configured to be inserted into a patient's body to allow a medical device to pass through the repositioning unit into the patient's body, the medical device extending through the proximal portion and distal portion of the repositioning unit; and (ii) a body which extends distally from the distal portion of the repositioning unit; wherein the proximal portion, distal portion, and body together form a lumen being configured to allow a medical device to pass through.

In a twelfth embodiment, based on the eleventh embodiment, the body is configured to be made of a puncture-resistant material.

In a thirteenth embodiment, based on the eleventh or twelfth embodiment, the body has a constant outer diameter.

In a fourteenth embodiment, based on the eleventh or twelfth embodiment, the body has an outer diameter that varies along the longitudinal length of the tubular body.

In a fifteenth embodiment, based on any of the eleventh through fourteenth embodiments, the body further comprises a wall, the wall having a discontinuity extending along at least a portion of the body.

In a sixteenth embodiment, based on the fifteenth embodiment, the discontinuity is a slot, the slot extending parallel to a central axis of the body.

In a seventeenth embodiment, a blood pump for percutaneous insertion into a patient's blood vessel is provided, including: (i) a catheter, and (ii) a pumping device attached to the catheter, the catheter extending along a longitudinal axis and having a distal end and a proximal end opposite the distal end along the longitudinal axis, the catheter comprising a first layer extending continuously longitudinally along the length of the catheter between the proximal end and the distal end of the catheter, the catheter further comprising a second layer extending longitudinally along at least a portion of the length of the catheter between the proximal and distal end of the catheter; wherein at least one of the first layer and the second layer is configured to be made of a puncture-resistant material.

In an eighteenth embodiment, based on the seventeenth embodiment, the first layer is made of a puncture-resistant material.

In a nineteenth embodiment, based on the seventeenth or eighteenth embodiment, the second layer is made of a puncture-resistant material.

In a twentieth embodiment, based on any of the seventeenth through nineteenth embodiments, the second layer extends continuously longitudinally along the length of the catheter.

In a twenty-first embodiment, based on any of the seventeenth through the twentieth embodiments, the second layer further comprises an outer diameter, the outer diameter decreasing continuously longitudinally along the length of the catheter.

In a twenty-second embodiment, based on the twenty-first embodiment, the outer diameter at the proximal end is greater than the outer diameter at the distal end.

In a twenty-third embodiment, based on any of the seventeenth through the twenty-second embodiments, the second layer extends from about 2 centimeters to about 5 centimeters longitudinally along the catheter.

In a twenty-fourth embodiment, based on the twenty-third embodiment, the second layer extends from about 3 centimeters to about 4 centimeters longitudinally along the catheter.

In a twenty-fifth embodiment, a method may be provided, that may include: (i) inserting a blood pump into a repositioning unit, the blood pump connected to a catheter, wherein at least a portion of the catheter has a puncture-resistant portion; and (ii) suturing the repositioning unit to a patient.

In a twenty-sixth embodiment, based on the twenty-fifth embodiment, the step of inserting a blood pump into a repositioning unit further comprises the step of inserting the catheter into a slot, the slot extending longitudinally along at least a portion of a tubular body made of a puncture-resistant material.

In a twenty-seventh embodiment, based on the twenty-fifth or twenty-sixth embodiment, the repositioning unit further comprises a proximal portion and a distal portion, the distal portion configured to be inserted into a patient's body to allow the blood pump to pass through the repositioning unit into the patient's body, the blood pump extending through the proximal portion and distal portion of the repositioning unit.

In a twenty-eighth embodiment, based on the twenty-seventh embodiment, the repositioning unit further comprises a tubular body which extends distally from the distal portion of the repositioning unit.

In a twenty-ninth embodiment, based on the twenty-eighth embodiment, the tubular body is made of a puncture-resistant material.

In a thirtieth embodiment, based on any of the twenty-fifth through the twenty-ninth embodiments, the catheter comprises a first layer and a second layer circumferentially surrounding the first layer, the second layer made of a puncture-resistant material.

In a thirty-first embodiment, an accessory for use with a blood pump system may be provided. The blood pump system may have a blood pump, a catheter attached to the blood pump, and a repositioning unit configured to receive at least a portion of the blood pump and/or catheter and to secure the blood pump system to the patient. The accessory may include: (i) a first end configured to be attached to a portion of a repositioning unit; and (ii) a second end, the second end connected to the first end by a body, the body surrounding at least a portion of the catheter; wherein when a puncture force is applied to the body, the body deflects the puncture force and the catheter is not punctured and/or damaged by the puncture force. In a thirty-second embodiment, an accessory for use with a blood pump system may be provided. The blood pump system may include a blood pump, a catheter attached to the blood pump, and a repositioning unit configured to receive at least a portion of the blood pump and/or catheter and to secure the blood pump system to the patient. The accessory may include: (i) a first end configured to be attached to a portion of a repositioning unit; and (ii) a second end, the second end connected to the first end by a body, the body surrounding at least a portion of the catheter; wherein the body is made of a puncture-resistant material.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Also, the phraseology and terminology used herein is for the purpose of description and should not be regarded as limiting. The use of "including", "comprising", or "having", "containing", "involving", and variations thereof herein, is meant to encompass the items listed thereafter and equivalents thereof as well as additional items.

In the claims, as well as in the specification above, all transitional phrases such as "comprising," "including", "carrying", "having", "containing", "involving", "holding", "composed of", and the like are to be understood to be open-ended, i.e., to mean including but not limited to. Only the transitional phrases "consisting of" and "consisting essentially of" shall be closed or semi-closed transitional phrases, respectively.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

## Claims

1. A blood pump system, comprising:
a catheter connected to the blood pump system, the catheter having a longitudinal axis and an outer wall extending circumferentially around and continuously along the longitudinal axis;
a repositioning unit, the repositioning unit having a proximal portion and a distal portion, at least a portion of the distal portion being configured to be implanted into a patient's vasculature; and
a tubular body with a distal end and a proximal end, the proximal end connected to the distal portion of the repositioning unit;
wherein the tubular body extends along at least a portion of the longitudinal axis of the catheter and is configured to surround at least a portion of the outer wall of the catheter.

2. The blood pump system of claim 1, wherein the tubular body is made of a material configured to be puncture resistant.

3. The blood pump system of claim 1 or 2, wherein the proximal end of the tubular body is removably attached to the distal portion of the repositioning unit.

4. The blood pump system of claim 1 or 2, wherein the tubular body is integrally formed with the distal portion of the repositioning unit.

5. The blood pump system of any one of claims 1 to 4, wherein the tubular body surrounds the entire circumference of the outer wall of the catheter.

6. The blood pump system of any one of claims 1 to 5, wherein the tubular body comprises a slot parallel to the longitudinal axis of the catheter.

7. The blood pump system of any one of claims 1 to 6, wherein the tubular body has a constant outer diameter.

8. The blood pump system of any one of claims 1 to 6, wherein the tubular body has an outer diameter that varies along the longitudinal axis of the catheter.

9. An accessory for use with a blood pump system, comprising:
a repositioning unit comprising a proximal portion and a distal portion, the distal portion configured to be inserted into a patient's body to allow a medical device to pass through the repositioning unit into the patient's body, the medical device extending through the proximal portion and distal portion of the repositioning unit; and
a body which extends distally from the distal portion of the repositioning unit;
wherein the proximal portion, distal portion, and body together form a lumen being configured to allow a medical device to pass through.

10. The accessory of claim 9, wherein the body is configured to be made of a puncture-resistant material.

11. The accessory of claim 9 or 10, wherein the body further comprises a wall, the wall having a discontinuity extending along at least a portion of the body.

12. A blood pump for percutaneous insertion into a patient's blood vessel, comprising:
a catheter, and
a pumping device attached to the catheter, the catheter extending along a longitudinal axis and having a distal end and a proximal end opposite the distal end along the longitudinal axis, the catheter comprising a first layer extending continuously longitudinally along the length of the catheter between the proximal end and the distal end of the catheter, the catheter further comprising a second layer extending longitudinally along at least a portion of the length of the catheter between the proximal and distal end of the catheter,
wherein at least one of the first layer and the second layer is configured to be made of a puncture-resistant material.

13. The blood pump of claim 12, wherein the second layer extends from about 2 centimeters to about 5 centimeters longitudinally along the catheter.

14. An accessory for use with a blood pump system, the blood pump system having a blood pump, a catheter attached to the blood pump, and a repositioning unit configured to receive at least a portion of the blood pump and/or catheter and to secure the blood pump system to the patient, the accessory comprising:
a first end configured to be attached to a portion of a repositioning unit;
a second end, the second end connected to the first end by a body, the body surrounding at least a portion of the catheter; and
wherein when a puncture force is applied to the body, the body deflects the puncture force and the catheter is not punctured and/or damaged by the puncture force.

15. An accessory for use with a blood pump system, the blood pump system having a blood pump, a catheter attached to the blood pump, and a repositioning unit configured to receive at least a portion of the blood pump and/or catheter and to secure the blood pump system to the patient, the accessory comprising:
a first end configured to be attached to a portion of a repositioning unit;
a second end, the second end connected to the first end by a body, the body surrounding at least a portion of the catheter; and
wherein the body is made of a puncture-resistant material.
